# EUROPÄISCHE PATENTSCHRIFT

(11) **EP 1 946 742 B1**
(45) Veröffentlichungstag und Bekanntmachung des Hinweises auf die Patenterteilung: **19.10.2011**
(21) Anmeldenummer: 08100402.0
(22) Anmeldetag: 14.01.2008
(51) Int. Cl.: A61K 8/06, A61K 8/34, A61K 8/36, A61K 8/89, A61Q 1/02

(54) **Kosmetische W/S-Emulsion mit 1,2-Decandiol**
Cosmetic W/S emulsion with 1.2 Decandiol
Emulsion eau/silicone pour la peau contenant du 1,2-décandiol

(30) Priorität: 19.01.2007 DE 102007003174
(43) Veröffentlichungstag der Anmeldung: 23.07.2008
(73) Patentinhaber: Beiersdorf AG, 20245 Hamburg (DE)
(72) Erfinder: Riedel, Heidi, 22083, Hamburg (DE); Hiddemann, Sarah, 22299, Hamburg (DE); Meiring, Uta, 21077, Hamburg (DE); Bleckmann, Andreas, 22926, Ahrensburg (DE)

(56) Entgegenhaltungen:
- EP-A- 0 584 692
- EP-A- 1 269 983
- EP-A- 1 872 770
- WO-A-2008/046791
- DE-A1- 10 223 694
- DE-A1- 10 341 179
- FR-A- 2 125 530

## Beschreibung

Die vorliegende Erfindung betrifft eine kosmetische Zubereitung enthaltend 1,2-Decandiol.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um Emulsionen über einen längeren Zeitraum stabil zu halten und eine Entmischung der Phasen zu verhindern, werden den Emulsionen sogenannte Emulgatoren zugesetzt. Bei Emulgatoren handelt es sich in der Regel um Moleküle mit einem polaren, hydrophilen Strukturelement und einem unpolaren, lipophilen Strukturelement. Ende der vierziger Jahre wurde ein System entwickelt, das die Auswahl von Emulgatoren erleichtern sollte. Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen öllösliche, hydrophobe Emulgatoren, Zahlen über 11 wasserlösliche, hydrophile.

Eine besondere Form der W/O-Emulsionen stellen Wasser-in-Silikonöl-Emulsionen (W/S-Emulsionen) dar. Bei ihnen besteht die Ölphase zum überwiegenden Teil aus Silikonölen (Cyclomethicon, Dimethicon). Diese Zubereitungen lassen sich nicht mehr mit herkömmlichen W/O-Emulgatoren herstellen. Es müssen spezielle Silikon-haltige Emulgatoren, beispielsweise Cetyl PEG/PPG-10/1 Dimethicon (Methylpolysiloxancetylpolysiloxanpoly-(oxyethylenoxypropylen)methylpolysiloxancopolymer, u.a. bei der Firma Degusa unter dem Handelsnamen Abil EM-90 erhältlich) eingesetzt werden. W/S-Emulsionen werden insbesondere bei schwierig zu formulierenden Produkten mit schwierig einzuarbeitenden Rohstoffen (beispielsweise Antitranspirant-Salzen) verwendet und ähneln in ihren sensorischen Eigenschaften den O/W-Emulsionen, sind also nicht so "fettig" wie herkömmliche W/O-Emulsionen.

Nachteilig am Stande der Technik ist jedoch der Umstand, dass W/S-Emulsionen sich nur schwierig konservieren lassen. Parabene und insbesondere das grenzflächenaktive Phenoxyethanol lassen sich allenfalls in (für eine wirkungsvolle Konservierung viel zu) geringen Mengen in W/S-Emulsionen einarbeiten. Wird die Konzentration dieser Konservierungsstoffe dagegen in für eine wirkungsvolle Konservierung notwendigen Konzentration eingesetzt, kommt es bei der Emulsion innerhalb kürzester Zeit zu Ölabscheidungen und Phasentrennungen. Dieser Effekt ist insbesondere bei dem Emulgator Cetyl PEG/PPG-10/1 Dimethicon zu beobachten.

Es war daher die Aufgabe der vorliegenden Erfindung, eine stabile W/S-Emulsion (insbesondere auf Basis des Emulgators Cetyl PEG/PPG-10/1 Dimethicon) zu entwickeln, die ausreichend vor dem mikrobiellen Befall und der mikrobiellen Zersetzung geschützt (also konserviert) ist.

Nachteilig am Stande der Technik ist ferner der Umstand, dass sich in herkömmliche W/S-Emulsionen (insbesondere auf Basis des Emulgators Cetyl PEG/PPG-10/1 Dimethicon) keine größeren Mengen an Pigmenten stabil einarbeiten lassen. Ab einem Pigmentgehalt von (je nach System) etwa 5 Gewichts-% kommt es bei diesen Emulsionen zu Ölabschidungen und Phasentrennung. Dies ist insbesondere dann von Nachteil, wenn auf der Basis von W/S-Emulsionen dekorative Kosmetika (z.B. Maskara, Foundations) entwickelt werden sollen.

Es war daher die Aufgabe der vorliegenden Erfindung, eine stabile W/S-Emulsion (insbesondere auf Basis des Emulgators Cetyl PEG/PPG-10/1 Dimethicon) zu entwickeln, die höhere Pigmentmengen (größer 5 Gewichts-%) vertragen. Insbesondere sollten gut und intensiv färbende kosmetische Zubereitungen (d.h. so genannte dekorative Kosmetika wie Foundations oder Maskara) auf der Basis einer W/S-Emulsion entwickelt werden

Überraschend gelöst werden die Aufgaben durch eine kosmetische Wasser-in -Silikonöl-Emulsion (W/S-Emulsion) enthaltend 1,2-Decandiol, **dadurch gekennzeichnet, dass** die Zubereitung als Emulgator Cetyl PEG/PPG-10/1 Dimethicon enthält.

Darüber hinaus zeigen die erfindungemäßen Zubereitungen überraschenderweise eine höhere Viskosität als Zubereitungen ohne 1,2-Decandiol. Dadurch kann die Menge an Verdickern und Stabilisatoren in der Zubereitung verringert werden. Die Verringerung der Menge an Verdickern und Stabilisatoren führt dazu, dass die Zubereitungen sich weniger stumpf auf der Haut anfühlen. Die Zubereitungen werden also für den Anwender sensorisch und optisch attraktiver.

Erfindungsgemäß werden unter W/S-Emulsion W/O-Emulsionen verstanden, die einen Emulgator auf Silikonbasis enthalten und deren Ölphase einen Silikonöl-Anteil (d.h. die Summe aus Cyclomethicon und Dimethicon und anderen) von mindestens 25 Gewichts-%, bevorzugt von 35-100 Gewichts-% bezogen auf das Gesamtgewicht der Ölphase aufweisen.

Erfindungsgemäß werden unter Verdickern und Stabilisatoren Wachse und Disteardimonium Hectorite verstanden.

Zwar kennt der Fachmann die DE 103 55 712 und DE 103 41 179, welche den Einsatz von 1,2-Decandiol in O/W-Foundations beschreiben, doch konnten diese Schriften nicht den Weg zur vorliegenden Erfindung weisen. Darüber hinaus waren dem Fachmann die EP 1269983, die DE 10223694, die EP 0584692 und die FR 2125530 bekannt, die ebenfalls nicht den Weg zur vorliegenden Erfindung weisen konnten.

Es ist erfindungsgemäß vorteilhaft, wenn die erfindungsgemäße Zubereitung 1,2-Decandiol in einer Konzentration von 0,01 bis 1,5 Gewichts-%, bevorzugt in einer Konzentration von 0,1 bis 1 Gewichts-% und besonders bevorzugt in einer Konzentration von 0,2-0,75 Gewichts-%, jeweils bezogen auf das Gesamtgewicht der Zubereitung, enthält.

Es ist erfindungsgemäß vorteilhaft, wenn die erfindungsgemäße Zubereitung Cetyl PEG/PPG-10/1 Dimethicon in einer Konzentration von 1 bis 7,5 Gewichts-%, bevorzugt in einer Konzentration von 2 bis 6 Gewichts-% und besonders bevorzugt in einer Konzentration von 3 bis 5 Gewichts-%, jeweils bezogen auf das Gesamtgewicht der Zubereitung, enthält. Cetyl PEG/PPG-10/1 Dimethicon kann beispielsweise unter dem Handelsnamen Abil EM-90 bei der Firma Degussa erworben werden.

Vorteilhafte Ausführungsformen der vorliegenden Erfindung sind **dadurch gekennzeichnet, dass** die Zubereitung Phenoxyethanol und/oder Parabene in einer Gesamtkonzentration von größer oder gleich 0,25 Gewichts-%, bezogen auf das Gesamtgewicht der Zubereitung, enthält.

Es ist erfindungsgemäß bevorzugt, wenn die erfindungsgemäße Emulsion Phenoxyethanol und Parabene enthält.

Erfindungsgemäß bevorzugt enthält die erfindungsgemäße Emulsion Phenoxyethanol in einer Konzentration von 0,01 bis 1 Gewichts-% und besonders bevorzugt in einer Konzentration von 0,5 bis 0,9 Gewichts-%, jeweils bezogen auf das Gesamtgewicht der Zubereitung.

Erfindungsgemäß bevorzugt enthält die erfindungsgemäße Emulsion Parabene in einer Konzentration von 0,01 bis 1 Gewichts-% und besonders bevorzugt in einer Konzentration von 0,4 bis 0,8 Gewichts-%, jeweils bezogen auf das Gesamtgewicht der Zubereitung. Erfindungsgemäß bevorzugte Parabene sind Methylparaben, Ethylparaben, Propylparaben und Butylparaben.

Im Bereich der kosmetischen Mittel sind zur Färbung des Produktes oder zur Färbung des zu behandelnden "Objektes" (Haut, Haare, Lippen) sowohl lösliche Farbmittel (im Rahmen der vorliegenden Beschreibung auch als Farbstoffe bezeichnet) und unlösliche Farbmittel (im Rahmen der vorliegenden Beschreibung auch als Farbpigmente (inklusive der Weißpigmente) oder Pigmente bezeichnet) zugelassen.

Erfindungsgemäß vorteilhafte Ausführungsformen der vorliegenden Erfindung sind **dadurch gekennzeichnet, dass** die Zubereitung Farbpigmente in einer Gesamtkonzentration von größer oder gleich 5 Gewichts-% und bevorzugt in einer Gesamtkonzentration von größer oder gleich 7 Gewichts-%, jeweils bezogen auf das Gesamtgewicht der Zubereitung, enthält.

Als erfindungsgemäße Pigmente können erfindungsgemäß vorteilhaft ein oder mehrere Verbindungen eingesetzt werden.

Bevorzugt im Sinne der vorliegenden Erfindung sind Pigmentmischungen aus Weiß-Pigmenten (z. B. Kaolin, Titandioxid oder Zinkoxid) und anorganischen Pigmenten (z. B. EisenoxidPigmente, Chromoxide), wobei die Pigmente beschichtet ("gecoatet") oder unbeschichtet vorliegen können.

Bei den Farbpigmenten kommen vorzugsweise die Eisenoxide zur Verwendung

Es kommen außerdem vorzugsweise Weißpigmente zur Anwendung, dies sind Pigmente, deren optische Wirkung vorwiegend auf nicht selektiver Lichtstreuung beruht (DIN 55944: 2003-11). Von den chemisch oft sehr ähnlichen Füllstoffen unterscheiden sich anorganische Weißpigmente vor allem durch ihre im allgemeinen höhere Brechzahl und, damit verbunden, ihr höheres Streuvermögen sowie nach DIN 55943: 2001-10 ihre Anwendung

Ideale Weißpigmente zeigen keine Absorption im Bereich des sichtbaren Lichts, dafür hohes Streuvermögen, das hohes Deckvermögen zur Folge hat. Das Streuvermögen ist umso größer, je größer die Differenz zwischen der Brechzahl des Weißpigmentes und der des umgebenden Mediums ist.

**Tabelle: Brechzahlen und Colour Index verschiedener Weißpigmente.**

| **Name** | **Formel** | **Brechzahl n** | **Colour Index** |
|---|---|---|---|
| *Weißpigmente* | | | |
| Titandioxid-Pigmente | TiO₂ | | PW 6, 77891 |
| - Anatas-Pigmente | | 2,55 | |
| - Rutil-Pigmente | | 2,75 | |
| Zinkoxid (Zinkweiß) | ZnO | 1,95 -2,1 | PW 4,77947 |
| *Füllstoffe* | | | |

Bevorzugt in der kosmetischen Zubereitung zum Einsatz kommen Titandioxide als Weißpigment.

Zusätzlich vorteilhaft können die kosmetischen Zubereitungen Effektpigmente enthalten, die neben Farbe eine zusätzliche Eigenschaft, wie z.B. Winkelabhängigkeit der Farbe (changieren, Flop), Glanz (nicht Oberflächenglanz) oder Textur, verleihen.

Die bedeutendste Gruppe der Effektpigmente stellen die Glanzpigmente dar, zu denen nach DIN 55944: 2003-11 die Metalleffektpigmente und die Perlglanzpigmente gehören (siehe nachstehende Tabelle). Einige spezielle Effektpigmente lassen sich diesen beiden Gruppen nicht zuordnen, z.B. plättchenförmiges Graphit, plättchenförmiges Eisenoxid und mikronisiertes Titandioxid. Letzteres erzeugt nicht einen Glanzeffekt, sondern einen winkelabhängigen Lichtstreueffekt.

Bei den Glanzpigmenten nach DIN 55943: 2001-10 handelt es sich vorwiegend plättchenförmige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für bevorzugte handelübliche Effektpigmente sind : Timiron® von Merck , Iriodin® von Merck (Perl- und Farbglanzpigmente für dekorative technische Anwendungen), Xirallic® von Merck (Farbintensive Kristalleffektpigmente)

Weiterhin können optional organische Farbstoffe zum Einsatz kommen. Dies sind im Anwendungsmedium praktisch unlösliche organische Farbmittel. Nach DIN 55944: 1990-04 können organische Pigmente nach chemischen Gesichtspunkten in Azopigmente und polycyclische Pigmente und nach koloristischen Gesichtspunkten in Bunt- oder Schwarzpigmente eingeteilt werden.

Polycyclische Pigemente umfassen nach Nach DIN 55944 p. P. alle organischen Nicht-Azopigmente, die durch aromatische und/oder heteroaromatische Ringsysteme charakterisiert sind.

Die erfindungsgemäßen kosmetischen Zubereitungen können ferner vorteilhaft Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) sowie Lauroyl Lysine, Polymethylsilesquioxane, Polymethylmethacrylate, Polymethylmethacrylate Crosspolymer, Nylon, Talkum, Mica,

Erfindungsgemäß besonders bevorzugt sind Lauroyllysine und/oder Polymethylsilesquioxane, als Sonsorikadditive zu wählen.

Feine Fältchen sieht man, weil sich die Lichtreflexion auf der Hautoberfläche von der in den Faltensenken unterscheidet. Es ist daher vorteilhaft im Sinne der vorliegenden Erfindung, lichtstreuende Pigmente einzusetzen, die die Faltentiefe optisch reduzieren, indem sie den Anteil an diffus reflektiertem Licht in der Falte reduzieren, wodurch die Haut glatter und natürlicher aussieht. Auf diese Weise läßt sich auch übermäßiger Glanz der Haut optisch reduzieren. Vorteilhaft zu diesem Zweck zu verwenden ist speziell beschichtetes Silica - z. B. ganz besonders vorteilhaft Silica LDP (Silica + Titanium Dioxide + Eisenoxid), welches unter dem Handelsnamen Ronasphere LDP von Merck erhältlich ist. Ist ist daher bevorzugt Silica LDP der dekorativen Zbereitung zu zusetzen.

Es ist erfindungsgemäß vorteilhaft, wenn die erfindungsgemäße Zubereitung Verdicker und Stabilisatoren in einer Konzentration von kleiner oder gleich 0,5 Gewichts-%, bezogen auf das Gesamtgewicht der Zubereitung, enthält.

Erfindungsgemäß bevorzugt ist die erfindungsgemäße Emulsion frei von Verdickern und Stabilisatoren.

Die Silikonölphase der erfindungsgemäßen Zubereitung enthält erfindungsgemäß vorteilhaft von 1 bis 40 Gewichts-%, bezogen auf das Gesamtgewicht der Ölphase Cyclomethicon.

Die Silikonölphase der erfindungsgemäßen Zubereitung enthält erfindungsgemäß vorteilhaft von 0 bis 15 Gewichts-%, bezogen auf das Gesamtgewicht der Ölphase Dimethicon.

Erfindungsgemäß vorteilhafte Ausführungsformen der vorliegenden Erfindung sind **dadurch gekennzeichnet, dass** die Zubereitung einen oder mehrere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol) (Handelsname: Tinosorb M); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat ist doch = Ethylhexylsalicylat, oder; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer (INCI: Polysilicone-15); 2,4-Bis-{[4-(2-ethyl-hexyloxy)-2-hydroxy]-phenyl}-6-(4-methoxyphenyl)-1 ,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; den Merocyaninverbindungen; in einer Konzentration von 0,01 bis 40 Gewichts-% bezogen auf das Gesamtgewicht der Zubereitung. Dabei sind Konzentrationen von 1 bis 20 Gewichts-%, bezogen auf das Gesamtgewicht der Zubereitung erfindungsgemäß bevorzugt.

Erfindungsgemäß vorteilhaft enthält die erfindungsgemäße Emulsion Feuchthaltemittel. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure, (2-Hydroxyethyl)harnstoff und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte wie besonders bevorzugt Salze wie Natriumchlorid und /oder Magnesiumsulfat, Selbstbräuner sowie ein oder mehrere Verdickungsmittel, welches oder welche vorteilhaft gewählt werden können aus der Gruppe Siliciumdioxid, Aluminiumsilikate, Polyacrylate, Polysaccharide bzw. deren Derivate, z. B. Hyaluronsäure, Xanthangummi, Hydroxypropylmethylcellulose, jeweils einzeln oder in Kombination.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP)

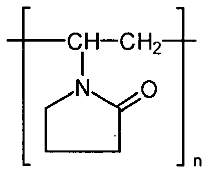

zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Insbesondere bevorzugt sind wasserlösliche Polymere wie Vinylpyrrolidon/Vinylacetat- (VP/VA-) Copolymere und Natriumpolystyrensulfonat.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin und/oder Licochalcon A.

Die kosmetischen Zubereitungen gemäß der Erfindung können kosmetische Hilfsstoffe enthalten, wie sie üblicherweise in solchen Zubereitungen verwendet werden, z. B. Konservierungshelfer, Komplexbildner, Bakterizide, Parfüme, Substanzen zum Verhindern oder Steigern des Schäumens, Farbstoffe.

Erfindungsgemäß ist auch das Verfahren zur Herstellung der erfindungsgemäßen Emulsion, welches dadurch gekennzeichnet ist, dass die Pigmente in der Ölphase, welche den Emulgator enthält, angeschlemmt werden. Im Anschluß wird die Wasserphase der Pigment/Ölphase zugesetzt und mittels Energieeintrag gleichmäßig vermengt. Wirkstoffe werden in einer extra Phase der Emulsion beigemengt. Das 1,2-Decandiol wird in einer gesonderten Phase aufgewärmt, verflüssigt und im flüssigen Zustand der Emulsion beigefügt.

Erfindungsgemäß ist auch die kosmetische Zubereitung die nach dem erfindungsgemäßen Verfahren hergestellt wird.

Erfindungsgemäß ist die Verwendung von 1,2-Decandiol zur Erhöhung der Stabilität von W/S-Emulsionen.

Erfindungsgemäß ist die Verwendung von 1,2-Decandiol zur Erhöhung Phasenstabilität von W/S-Emulsionen.

Erfindungsgemäß ist die Verwendung von 1,2-Decandiol zur Erhöhung der mikrobiellen Stabilität von W/S-Emulsionen.

Erfindungsgemäß ist die Verwendung von 1,2-Decandiol zur Erhöhung der Viskosität von Wasser- in Silikonöl-Emulsionen.

### Vergleichsversuche

| **W/S Emulsion** | **1** | **2** | **3** | **4** | **5** | **6** |
|---|---|---|---|---|---|---|
| Cetyl PEG/PPG-10/1 Dimethicone | 4 | 4 | 4 | 4 | 4 | 4 |
| Silikonöl (Cyclomethicon & Dimethicon) | 20 | 20 | 20 | 20 | 20 | 20 |
| polare / mittelpolare Öle | 5,5 | 5,5 | 5,5 | 5,5 | 5,5 | 5,5 |
| Disteardimonium Hectorite | 0,25 | 0,25 | 0,25 | 0,25 | 0,25 | 0,25 |
| Propylene Carbonate | 0,06 | 0,06 | 0,06 | 0,06 | 0,06 | 0,06 |
| Farbpigmente (Titandioxid + Eisenoxide) | 6 | 6 | 6 | 6 | 6 | 6 |
| Silica + Titandioxid + Eisenoxid | 3 | 3 | 3 | 3 | 3 | 3 |
| Puderrohstoffe (Lauroyl Lysine, Nylon-12, Talkum, Silica) | 6 | 6 | 6 | 6 | 6 | 6 |
| Moisturizer (Polyole wie Glycerin) | 5 | 5 | 5 | 5 | 5 | 5 |
| Parfum | 0,3 | 0,3 | 0,3 | 0,3 | 0,3 | 0,3 |
| Natrium Chlorid | 2 | 2 | 2 | 2 | 2 | 2 |
| *Methylparaben* | *0,2* | *0,36* | | | *0,16* | *0,1* |
| *Propylparaben* | *0,1* | *0,12* | | | *0,02* | *0,014* |
| *Phenoxyethanol* | *0,5* | *1,22* | | | *0,72* | *0,5* |
| ***1,2-Decandiol*** | | | ***0,3*** | ***0,3*** | ***0,3*** | ***0,3*** |
| *1,2-Hexanediol* | *0,3* | *0,3* | *0,75* | *0,75* | *0,3* | *0,75* |
| Wasser | ad. 100 | ad. 100 | ad. 100 | ad. 100 | ad. 100 | ad. 100 |
| **Stabilität 4 Monate bei 40°C** | **Ölabscheidung** | **Ölabscheidung** | ***Stabil*** | ***Stabil*** | ***Stabil*** | ***Stabil*** |
| **Viskosität [mPa/s] 1-4 Monate** | **1700 - 2200** | **2400 - 3000** | **3600 - 3900** | **3200 - 3500** | **3000 - 3700** | **3300 - 4200** |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Foundation

### W/S Emulsionen

| | **1** | **2** | **3** | **4** | **5** | **6** | **7** |
|---|---|---|---|---|---|---|---|
| Cetyl PEG/PPG -10/1 Dimethicon | | 3,00 | | 4,00 | 3,00 | 4,00 | 4,00 |
| Polyglyceryl-2-dipolyhydroxystearat | 5,00 | | | | | | |
| PEG-30-dipolyhydroxystearat | | | 5,00 | | | | |
| Ethylhexyl Methoxycinnamat | | 3,50 | | 10,00 | | | |
| Titandioxid T 805 | | 1,50 | | | 1,00 | 0,50 | |
| Titandioxid MT-100Z | 1,00 | | | 3,00 | 1,00 | | |
| Dicaprylylether | | | 3,50 | | 1,00 | | |
| Dicaprylyl Carbonate | | | | | | 4,00 | 4,00 |
| Squalane | | 5,00 | | | | | |
| Dimethicon | 5,00 | 4,50 | 1,00 | 3,00 | 2,00 | 4 | 5 |
| Cyclomethicon | 5,00 | 15,0 | 5,00 | 10,50 | 25,00 | 12 | 10 |
| Dimethicon / Vinyl Dimethicon Crosspolymer | 4,00 | | | | | | 1,00 |
| Shea Butter | 1,50 | 0,5 | | 1,00 | 1,00 | | 2,00 |
| Methyl Methaacrylate Crosspolymer | 1,00 | | | | | | 1,00 |
| Nylon -12 | 2,00 | | | | | 2,00 | |
| Polymethylsilesquioxane | | 2,00 | | 2,00 | 1,00 | 1,00 | |
| Lauroyl Lysine | | 3,00 | | 2,00 | 2,00 | | 1,00 |
| Titandioxid (CI 77891) | 9,00 | 6,00 | 7,00 | 6,00 | 4,50 | 3,00 | 10,00 |
| Pigmente (CI77492, 77491,77499,77007) | 2,0 | 3,0 | 2,6 | 4,0 | 1,5 | 2,5 | 3,0 |
| Beschichtetes Silica ² | | 0,50 | | 1,00 | 0,50 | | 1,0 |
| Effektpigmente (z.B. beschichtetes Mica)³ | | 1,00 | 0,50 | | | | 0,27 |
| Glycerin | 3,00 | 7,50 | | 7,50 | 5,00 | | 10,00 |
| Butylenglycol | 7,00 | 5,00 | | | | 7,00 | |
| Vitamin A Palmitat | 0,50 | 0,1 | 0,25 | | | | 1,00 |
| Vitamin C Phosphat | 0,1 | 0,05 | 0,5 | 0,1 | 0,25 | 0,5 | 0,1 |
| Hyaluronsäure | 0,01 | 0,05 | 0,50 | 0,03 | 0,35 | 1,0 | 0,1 |
| Gingko Biloba | 0,1 | 0,2 | 1,50 | 0,25 | 0,10 | 2,00 | 1,00 |
| Natrium Polystyrene Sulfonate | 0,2 | 0,5 | 0,25 | 0,1 | 0,2 | 2,0 | 1,0 |
| VPNA Copolymer | 0,5 | 0,2 | 0,25 | 1,0 | 0,2 | 1,0 | 2,0 |
| PVP Hexadecen Copolymer | 0,5 | | | | 0,10 | | 1,00 |
| Magnesium Sulfate | | | | 1,00 | 1,00 | | |
| Natrium Chlorid | 2,00 | 1,00 | 2,00 | | | 2,00 | 2,00 |
| Propylene Carbonate | 0,12 | 0,12 | 0,12 | 0,12 | 0,12 | 0,12 | 0,12 |
| Disteardimonium Hectorite | 0,5 | 0,5 | 0,5 | 0,5 | 0,5 | 0,5 | 0,5 |
| 1,2 Decandiol | 0,30 | 0,30 | 0,30 | 0,30 | 0,30 | 0,30 | 0,30 |
| Parabene | 0,2 | 0,15 | 0,25 | | 0,50 | 0,2 | 0,70 |
| Phenoxyethanol | 0,5 | 0,70 | 0,5 | | 0,50 | 0,25 | 0,90 |
| 1,2-Hexandiol | | | | 0,70 | | 0,75 | |
| EDTA oder Tetra Sodium Iminodisuccinate | | 0,20 | 0,35 | 0,50 | 0,02 | | 0,03 |
| Ethanol | 3,00 | 3,00 | 2,00 | 1,00 | 3,00 | 5,00 | 2,50 |
| Parfüm | 0,20 | 0,20 | | | | 0,30 | 0,40 |
| Wasser | ad 100 | ad 100 | ad 100 | ad 100 | ad 100 | ad 100 | ad 100 |

| | | | | | | | |
|---|---|---|---|---|---|---|---|
| ^{2 z.B.} Ronasphere LDP von Merck (Silica + Titanium Dioxide + Iron Oxides) ^{3 z.B..} Timiron von Merck (Mica & CI 77891) | | | | | | | |

## Patentansprüche

1. Kosmetische Wasser-in -Silikonöl-Emulsion enthaltend 1,2-Decandiol, **dadurch gekennzeichnet, dass** die Zubereitung als Emulgator Cetyl PEG/PPG-10/1 Dimethicon enthält,

2. Kosmetische Emulsion nach einem der vorhergehenden Ansprüche, **dadurch gekennzeichnet, dass** die Zubereitung 1,2-Decandiol in einer Konzentration von 0,01 bis 1,5 Gewichts-%, bezogen auf das Gesamtgewicht der Zubereitung, enthält.

3. Kosmetische Emulsion nach einem der vorhergehenden Ansprüche, **dadurch gekennzeichnet, dass** die Zubereitung Cetyl PEG/PPG-10/1 Dimethicon in einer Konzentration von 1 bis 7,5 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung Phenoxyethanol und/oder Parabene in einer Gesamtkonzentration von größer oder gleich 0,25 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung Pigmente in einer Gesamtkonzentration von größer oder gleich 5 Gewichts-%, bezogen auf das Gesamtgewicht der Zubereitung, enthält.

6. Kosmetische Zubereitung nach einem der vorhergehenden Ansprüche, **dadurch gekennzeichnet, dass** die Zubereitung Verdicker und Stabilisatoren in einer Konzentration von kleiner oder gleich 0,5 Gewichts-%, bezogen auf das Gesamtgewicht der Zubereitung, enthält.

7. Verfahren zur Herstellung einer Wasser-in-Silikonöl Emulsion nach einem der vorhergehenden Ansprüche, **dadurch gekennzeichnet, dass**
a) die Pigmente in der Ölphase, welche den Emulgator Cetyl PEGIPPG-10/1 Dimethicone enthält, angeschlemmt werden,
b) im Anschluß die Wasserphase der Pigment/Ölphase zugesetzt und mittels Energieeintrag gleichmäßig vermengt wird,
c) ggf. vorhandene Wirkstoffe in einer extra Phase der Emulsion beigemengt werden und
d) das 1,2-Decandiol in einer gesonderten Phase aufgewärmt, verflüssigt und im flüssigen Zustand der Emulsion beigefügt wird.

8. Kosmetische Zubereitung hergestellt nach einem Verfahren nach Anspruch 7.

## Claims

1. Cosmetic water-in-silicone oil emulsion comprising 1,2-decanediol, **characterized in that** the preparation comprises cetyl PEG/PPG-10/1 dimethicone as emulsifier.

2. Cosmetic emulsion according to Claim 1, **characterized in that** the preparation comprises 1,2-decanediol in a concentration of from 0.01 to 1.5% by weight, based on the total weight of the preparation

3. Cosmetic emulsion according to one of the preceding claims, **characterized in that** the preparation comprises cetyl PEG/PPG-10/1 dimethicone in a concentration of from 1 to 7.5% by weight, based on the total weight of the preparation.

4. Cosmetic preparation according to one of the preceding claims, **characterized in that** the preparation comprises phenoxyethanol and/or parabens in a total concentration of greater than or,equal to 0.25% by weight, based on the total weight of the preparation.

5. Cosmetic preparation according to one of the preceding claims, **characterized in that** the preparation comprises pigments in a total concentration of greater than or equal to 5% by weight, based on the total weight of the preparation.

6. Cosmetic preparation according to one of the preceding claims, **characterized in that** the preparation comprises thickeners and stabilizers in a concentration of less than or equal to 0.5% by weight, based on the total weight of the preparation.

7. Process for the preparation of a water-in-silicone oil emulsion according to one of the preceding claims, **characterized in that**
a) the pigments are slurried in the oil phase, which comprises the emulsifier cetyl PEG/PPG-10/1 dimethicone,
b) then the water phase is added to the pigment/oil phase and uniformly blended by introducing energy,
c) if appropriate, present active ingredients are admixed in an extra phase of the emulsion,
and
d) the 1,2-decanediol is heated in a separate phase, liquefied and added in the liquid state of the emulsion.

8. Cosmetic preparation prepared by a process according to Claim 7.

## Revendications

1. Emulsion cosmétique eau-dans-huile de silicone, contenant du 1,2-décanediol, **caractérisée en ce que** la composition contient du Cetyl PEG/PPG-10/1 Dimethicon comme émulsifiant.

2. Emulsion cosmétique selon la revendication 1, **caractérisée en ce que** la composition contient le 1,2-décanediol en une concentration de 0,01 à 1,5% en poids par rapport au poids total de la composition.

3. Emulsion cosmétique selon l'une quelconque des revendications précédentes, **caractérisée en ce que** la composition contient le Cetyl PEG/PPG-10/1 Dimethicon en une concentration de 1 à 7,5% en poids par rapport au poids total de la composition.

4. Composition cosmétique selon l'une quelconque des revendications précédentes, **caractérisée en ce que** la composition contient du phénoxyéthanol et/ou des parabènes en une concentration totale supérieure ou égale à 0,25% en poids par rapport au poids total de la composition.

5. Composition cosmétique selon l'une quelconque des revendications précédentes, **caractérisée en ce que** la composition contient des pigments en une concentration totale supérieure ou égale à 5% en poids par rapport au poids total de la composition.

6. Composition cosmétique selon l'une quelconque des revendications précédentes, **caractérisée en ce que** la composition contient des épaississants et des stabilisateurs en une concentration inférieure ou égale à 0,5% en poids par rapport au poids total de la composition.

7. Procédé pour la préparation d'une émulsion eau-dans-huile de silicone selon l'une quelconque des revendications précédentes, **caractérisé en ce que**
a) les pigments sont gâchés dans la phase huileuse, qui contient l'émulsifiant Cetyl PEG/PPG-10/1 Dimethicon,
b) ensuite, la phase aqueuse est ajoutée à la phase de pigment/huile et mélangée régulièrement par introduction d'énergie,
c) les substances actives le cas échéant présentes sont mélangées dans une phase supplémentaire de l'émulsion et
d) le 1,2-décanediol est chauffé dans une phase séparée, liquéfié et ajouté à l'émulsion sous forme liquide.

8. Composition cosmétique préparée selon un procédé selon la revendication 7.
